# EUROPEAN PATENT APPLICATION

(11) **EP 4 425 304 A2**
(43) Date of publication of application: **04.09.2024**
(21) Application number: 24154466.7
(22) Date of filing: 29.01.2024
(51) Int. Cl.: G06F 3/01, G01S 13/00, G01S 13/04, G01S 13/06, G01S 13/88

(54) **RADAR-ASSISTED THREE-DIMENSIONAL (3D) DETECTION FOR NEAR-EYE DISPLAY DEVICES**

(30) Priority: 28.02.2023 US 202318115234
(71) Applicant: Meta Platforms Technologies, LLC, Menlo Park, CA 94025 (US)
(72) Inventor: Yang, Yin, Menlo Park (US)
(74) Representative: Murgitroyd & Company

(57) **Abstract**

An augmented reality (AR) / virtual reality (VR) near-eye display device employs radar technology to detect gestures or obtain depth information associated with a real scene around the user. An on-board radar sub-system detects motion such as hand or finger gestures to be used as input related to different functions of the near-eye display device. The radar sub-system may also be used to activate an on-board camera. The radar sub-system may obtain depth information associated with the real scene around the user, and a 2D image of the real scene captured by the camera may be processed to reconstruct a 3D image of the real scene. Bio-signal(s) of the user such as breath rate, heart rate, tremors, twitching, etc. may also be captured by the radar sub-system alone or in combination with the camera and be used for health monitoring.

## Description

### TECHNICAL FIELD

This patent application relates generally to near-eye display devices, and in particular, gesture and three-dimensional (3D) scene capture and detection through a radar and/or a camera on a near-eye display device.

### BACKGROUND

With recent advances in technology, prevalence and proliferation of content creation and delivery has increased greatly in recent years. In particular, interactive content such as virtual reality (VR) content, augmented reality (AR) content, mixed reality (MR) content, and content within and associated with a real and/or virtual environment (e.g., a "metaverse") has become appealing to consumers.

To facilitate delivery of this and other related content, service providers have endeavored to provide various forms of wearable display systems. One such example may be a head-mounted display (HMD) device, such as a wearable eyewear, a wearable headset, or eyeglasses. In some examples, the head-mounted display (HMD) device may project or direct light to may display virtual objects or combine images of real objects with virtual objects, as in virtual reality (VR), augmented reality (AR), or mixed reality (MR) applications. For example, in an AR system, a user may view both images of virtual objects (e.g., computer-generated images (CGIs)) and the surrounding environment. Head-mounted display (HMD) devices may also present interactive content, where a user's (wearer's) gaze may be used as input for the interactive content.

### SUMMARY

In accordance with a first aspect, there is provided a detection system for a near-eye display device, comprising:
a radar sub-system configured to detect a gesture, wherein the radar sub-system comprises at least one antenna, a radio frequency (RF) front end, RF processing circuitry, or digital processing circuitry;
an image sensor configured to capture a two-dimensional (2D) image of the detected gesture; and
a processor communicatively coupled to the radar sub-system and the image sensor, the processor to:
   activate the image sensor upon detection of the gesture by the radar sub-system; and
   provide an input to an application executed at the near-eye display device based on the detected gesture.

The gesture may comprise at least one of a hand gesture, a finger gesture, or a body gesture.

The radar sub-system may be configured to detect the gesture directly or indirectly from a nearby reflective surface.

An antenna of the radar sub-system may be positioned downward from a head of a user and at a predefined angle to detect the gesture while the user is in a natural pose.

The image sensor may comprise an outward-facing camera configured to capture the 2D image of the gesture directly or indirectly from a nearby reflective surface.

The processor may be further configured to:
compare an output of the radar sub-system and an output of the image sensor so as to enhance an accuracy of the detected gesture.

The radar sub-system may be a millimeter-wave radar sub-system.

In accordance with a second aspect, there is provided a detection system for a near-eye display device, comprising:
a radar sub-system configured to capture a depth characteristic of a scene around a user wearing the near-eye display device, wherein the radar sub-system comprises at least one antenna, a radio frequency (RF) front end, RF processing circuitry, or digital processing circuitry;
an image sensor configured to capture a two-dimensional (2D) image of the scene; and
a processor communicatively coupled to the radar sub-system and the image sensor, the processor to:
   receive the 2D image from the image sensor and the depth characteristic from the radar sub-system; and
   reconstruct a three-dimensional (3D) image of the scene based on the 2D image and the depth characteristic.

The depth characteristic may comprise depth information associated with at least one object in the scene.

The radar sub-system may comprise a millimeter-wave radar sub-system that provides millimeter level spatial resolution.

The radar sub-system may employ 58.0 GHz to 63.5 GHz frequency range with a bandwidth of 5.5 GHz.

The processor may be further configured to provide the reconstructed 3D image of the scene to a display of the near-eye display device.

In accordance with a third aspect, there is provided a detection system for a near-eye display device, comprising:
a radar sub-system configured to detect a bio-signal of a user wearing the near-eye display device, wherein the radar sub-system comprises at least one antenna, a radio frequency (RF) front end, RF processing circuitry, or digital processing circuitry; and
a processor communicatively coupled to the radar sub-system, the processor being configured to:
   provide the detected bio-signal to a health monitoring server.

The bio-signal may comprise at least one of a breath rate, a heart rate, a twitch or a tremor.

The detection system may further comprise:
an image sensor configured to capture an image, wherein the processor is further configured to:
activate the image sensor upon detection of the bio-signal by the radar sub-system; and
instruct the image sensor to capture the image.

The image sensor may comprise an outward-facing camera configured to capture the image directly or indirectly from a nearby reflective surface.

The captured image may comprise a scene around the user or an image of the user.

An antenna of the radar sub-system may be positioned downward from a head of the user and at a predefined angle to detect the bio-signal while the user is in a natural pose.

An antenna of the radar sub-system may be positioned to detect the bio-signal indirectly from a nearby reflective surface.

The radar sub-system may comprise a millimeter-wave radar sub-system.

It will be appreciated that any features described herein as being suitable for incorporation into one or more aspects are intended to be generalizable across any and all aspects described herein. Other aspects of the present disclosure can be understood by those skilled in the art in light of the description, the claims and the drawings. The foregoing general description and the following detailed description are exemplary and explanatory only and are not restrictive of the claims.

### BRIEF DESCRIPTION OF DRAWINGS

Features of the present disclosure are illustrated by way of example and not limited in the following figures, in which like numerals indicate like elements. One skilled in the art will readily recognize from the following that alternative examples of the structures and methods illustrated in the figures can be employed without departing from the principles described herein.
Figure 1 illustrates a block diagram of an artificial reality system environment including a near-eye display device, according to an example.
Figure 2 illustrates a perspective view of a near-eye display device in the form of a head-mounted display (HMD) device, according to an example.
Figures 3A and 3B illustrate a perspective view and a top view of a near-eye display device in the form of a pair of glasses, according to an example.
Figure 4 illustrates direct or indirect gesture detection through a radar on a near-eye display device, according to examples.
Figure 5A illustrates enhanced gesture detection through a combination of a radar and a camera, and activation of a gesture detecting camera through a radar in a near-eye display device, according to examples.
Figure 5B illustrates 3D reconstruction of a real scene through a combination of a camera and a radar in a near-eye display device, according to examples.
Figure 5C illustrates bio-signal detection by a radar in a near-eye display device for camera activation or health monitoring, according to examples.
Figure 6 illustrates a flow diagram for a method of gesture and 3D scene capture and detection through a radar and/or a camera on a near-eye display device, according to some examples.

### DETAILED DESCRIPTION

For simplicity and illustrative purposes, the present application is described by referring mainly to examples thereof. In the following description, numerous specific details are set forth in order to provide a thorough understanding of the present application. It will be readily apparent, however, that the present application may be practiced without limitation to these specific details. In other instances, some methods and structures readily understood by one of ordinary skill in the art have not been described in detail so as not to unnecessarily obscure the present application. As used herein, the terms "a" and "an" are intended to denote at least one of a particular element, the term "includes" means includes but not limited to, the term "including" means including but not limited to, and the term "based on" means based at least in part on.

As used herein, "radar" refers to an electromagnetic sensor for detecting, locating, tracking, and recognizing objects of various kinds at a distance by transmitting electromagnetic energy toward the objects and observing the echoes returned from the objects. A "real scene" refers to a physical environment around a user wearing augmented reality (AR) / virtual reality (VR) display device such as a near-eye display device. A "virtual scene" refers to a computer generated image of a scene and/or objects that is displayed through the near-eye display device to the user. A "bio-signal" refers to any biometric characteristic of a person that may be detected through a radar, a camera, or other device. Examples of bio-signals include, but are not limited to, hear rate, breath rate, skin movements, muscle contractions, head shaking, hand or finger tremors, and similar ones.

Augmented reality (AR) / virtual reality (VR) near-eye display devices, also referred to as smart glasses, commonly employ capacitive touch technology as a primary user input. Thus, a user needs to raise and hold their hand to the glass temple arm and swipe fingers in close contact with the smart glass. Supported gestures using this technique are limited, and the posture may be deemed unnatural for some users. While some smart glasses may be paired with a wrist input device employing tactile input or electromyography technology, such accessory devices may not be available to all the users.

In some examples of the present disclosure, a wearable device such as an augmented reality (AR) / virtual reality (VR) near-eye display device may employ radar technology to detect gestures or obtain depth information associated with a real scene around the user. An on-board radar sub-system may use time difference, frequency shift, etc. to detect motion such as hand or finger gestures, which may be used as input related to different functions of the near-eye display device. Due to low power consumption, the radar sub-system may be maintained in active mode for longer periods and also be used to activate an on-board camera. In some cases, the radar sub-system may obtain depth information associated with the real scene around the user, and a two-dimensional (2D) image of the real scene may be captured by the on-board camera. The radar sub-system and camera outputs may then be combined to reconstruct a three-dimensional (3D) image of the real scene. In further examples, bio-signal(s) of the user such as breath rate, heart rate, tremors, twitching, etc. may also be captured by the radar sub-system alone or in combination with the camera and be used for health monitoring.

While some advantages and benefits of the present disclosure are apparent, other advantages and benefits may include providing near-eye display devices with a wide range of gesture detection and 3D scene reconstruction with low power consumption, health monitoring, and enhanced accuracy in input processing.

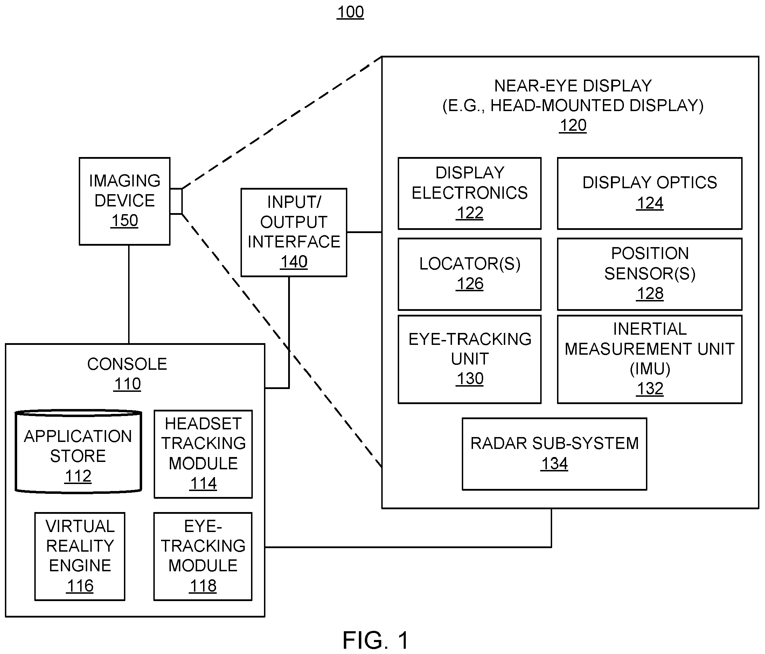

Figure 1 illustrates a block diagram of an artificial reality system environment 100 including a near-eye display device, according to an example. As used herein, a "near-eye display device" may refer to a device (e.g., an optical device) that may be in close proximity to a user's eye. As used herein, "artificial reality" may refer to aspects of, among other things, a "metaverse" or an environment of real and virtual elements and may include use of technologies associated with virtual reality (VR), augmented reality (AR), and/or mixed reality (MR). As used herein a "user" may refer to a user or wearer of a "near-eye display device."

As shown in Figure 1, the artificial reality system environment 100 may include a near-eye display device 120, an optional external imaging device 150, and an optional input/output interface 140, each of which may be coupled to a console 110. The console 110 may be optional in some instances as the functions of the console 110 may be integrated into the near-eye display device 120. In some examples, the near-eye display device 120 may be a head-mounted display (HMD) that presents content to a user.

In some instances, for a near-eye display system, it may generally be desirable to expand an eye box, reduce display haze, improve image quality (e.g., resolution and contrast), reduce physical size, increase power efficiency, and increase or expand field of view (FOV). As used herein, "field of view" (FOV) may refer to an angular range of an image as seen by a user, which is typically measured in degrees as observed by one eye (for a monocular head-mounted display (HMD)) or both eyes (for binocular head-mounted displays (HMDs)). Also, as used herein, an "eye box" may be a two-dimensional box that may be positioned in front of the user's eye from which a displayed image from an image source may be viewed.

In some examples, in a near-eye display system, light from a surrounding environment may traverse a "see-through" region of a waveguide display (e.g., a transparent substrate) to reach a user's eyes. For example, in a near-eye display system, light of projected images may be coupled into a transparent substrate of a waveguide, propagate within the waveguide, and be coupled or directed out of the waveguide at one or more locations to replicate exit pupils and expand the eye box.

In some examples, the near-eye display device 120 may include one or more rigid bodies, which may be rigidly or non-rigidly coupled to each other. In some examples, a rigid coupling between rigid bodies may cause the coupled rigid bodies to act as a single rigid entity, while in other examples, a non-rigid coupling between rigid bodies may allow the rigid bodies to move relative to each other.

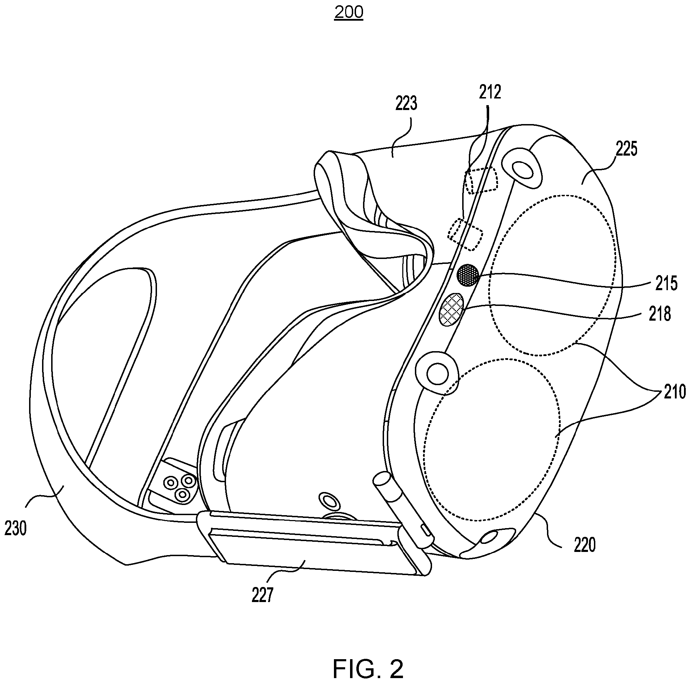

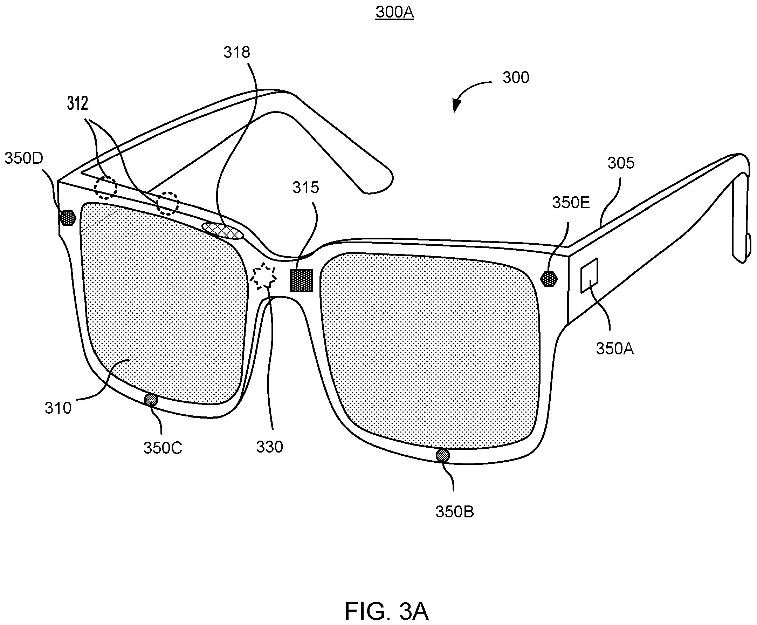

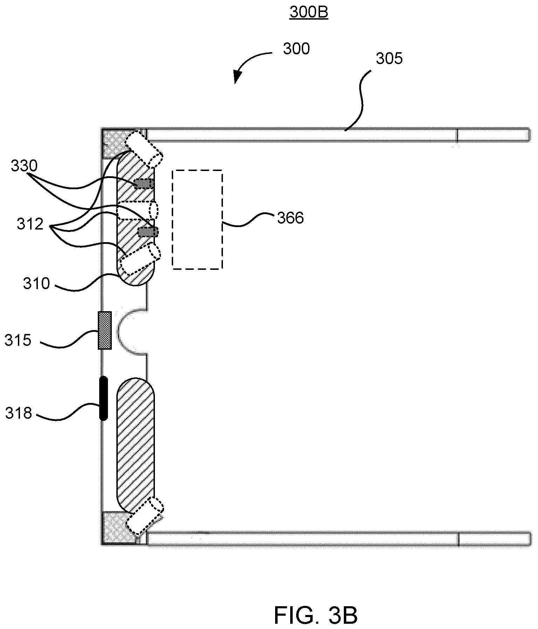

In some examples, the near-eye display device 120 may be implemented in any suitable form-factor, including a head-mounted display (HMD), a pair of glasses, or other similar wearable eyewear or device. Examples of the near-eye display device 120 are further described below with respect to Figures 2 and 3. Additionally, in some examples, the functionality described herein may be used in a head-mounted display (HMD) or headset that may combine images of an environment external to the near-eye display device 120 and artificial reality content (e.g., computer-generated images). Therefore, in some examples, the near-eye display device 120 may augment images of a physical, real-world environment external to the near-eye display device 120 with generated and/or overlaid digital content (e.g., images, video, sound, etc.) to present an augmented reality to a user.

In some examples, the near-eye display device 120 may include any number of display electronics 122, display optics 124, and an eye tracking unit 130. In some examples, the near-eye display device 120 may also include one or more locators 126, one or more position sensors 128, an inertial measurement unit (IMU) 132, and a radar subs-system 134. In some examples, the near-eye display device 120 may omit any of the eye tracking unit 130, the one or more locators 126, the one or more position sensors 128, and the inertial measurement unit (IMU) 132, or may include additional elements.

In some examples, the display electronics 122 may display or facilitate the display of images to the user according to data received from, for example, the optional console 110. In some examples, the display electronics 122 may include one or more display panels. In some examples, the display electronics 122 may include any number of pixels to emit light of a predominant color such as red, green, blue, white, or yellow. In some examples, the display electronics 122 may display a three-dimensional (3D) image, e.g., using stereoscopic effects produced by two-dimensional panels, to create a subjective perception of image depth.

In some examples, the near-eye display device 120 may include a projector (not shown), which may form an image in angular domain for direct observation by a viewer's eye through a pupil. The projector may employ a controllable light source (e.g., a laser source) and a micro-electromechanical system (MEMS) beam scanner to create a light field from, for example, a collimated light beam. In some examples, the same projector or a different projector may be used to project a fringe pattern on the eye, which may be captured by a camera and analyzed (e.g., by the eye tracking unit 130) to determine a position of the eye (the pupil), a gaze, etc.

In some examples, the display optics 124 may display image content optically (e.g., using optical waveguides and/or couplers) or magnify image light received from the display electronics 122, correct optical errors associated with the image light, and/or present the corrected image light to a user of the near-eye display device 120. In some examples, the display optics 124 may include a single optical element or any number of combinations of various optical elements as well as mechanical couplings to maintain relative spacing and orientation of the optical elements in the combination. In some examples, one or more optical elements in the display optics 124 may have an optical coating, such as an anti-reflective coating, a reflective coating, a filtering coating, and/or a combination of different optical coatings.

In some examples, the display optics 124 may also be designed to correct one or more types of optical errors, such as two-dimensional optical errors, three-dimensional optical errors, or any combination thereof. Examples of two-dimensional errors may include barrel distortion, pincushion distortion, longitudinal chromatic aberration, and/or transverse chromatic aberration. Examples of three-dimensional errors may include spherical aberration, chromatic aberration field curvature, and astigmatism.

In some examples, the one or more locators 126 may be objects located in specific positions relative to one another and relative to a reference point on the near-eye display device 120. In some examples, the optional console 110 may identify the one or more locators 126 in images captured by the optional external imaging device 150 to determine the artificial reality headset's position, orientation, or both. The one or more locators 126 may each be a light-emitting diode (LED), a corner cube reflector, a reflective marker, a type of light source that contrasts with an environment in which the near-eye display device 120 operates, or any combination thereof.

In some examples, the external imaging device 150 may include one or more cameras, one or more video cameras, any other device capable of capturing images including the one or more locators 126, or any combination thereof. The optional external imaging device 150 may be configured to detect light emitted or reflected from the one or more locators 126 in a field of view of the optional external imaging device 150.

In some examples, the one or more position sensors 128 may generate one or more measurement signals in response to motion of the near-eye display device 120. Examples of the one or more position sensors 128 may include any number of accelerometers, gyroscopes, magnetometers, and/or other motion-detecting or error-correcting sensors, or any combination thereof.

In some examples, the inertial measurement unit (IMU) 132 may be an electronic device that generates fast calibration data based on measurement signals received from the one or more position sensors 128. The one or more position sensors 128 may be located external to the inertial measurement unit (IMU) 132, internal to the inertial measurement unit (IMU) 132, or any combination thereof. Based on the one or more measurement signals from the one or more position sensors 128, the inertial measurement unit (IMU) 132 may generate fast calibration data indicating an estimated position of the near-eye display device 120 that may be relative to an initial position of the near-eye display device 120. For example, the inertial measurement unit (IMU) 132 may integrate measurement signals received from accelerometers over time to estimate a velocity vector and integrate the velocity vector over time to determine an estimated position of a reference point on the near-eye display device 120. Alternatively, the inertial measurement unit (IMU) 132 may provide the sampled measurement signals to the optional console 110, which may determine the fast calibration data.

The eye tracking unit 130 may include one or more eye tracking systems. As used herein, "eye tracking" may refer to determining an eye's position or relative position, including orientation, location, and/or gaze of a user's eye. In some examples, an eye tracking system may include an imaging system that captures one or more images of an eye and may optionally include a light emitter, which may generate light (e.g., a fringe pattern) that is directed to an eye such that light reflected by the eye may be captured by the imaging system (e.g., a camera). In other examples, the eye tracking unit 130 may capture reflected radio waves emitted by a miniature radar unit. These data associated with the eye may be used to determine or predict eye position, orientation, movement, location, and/or gaze.

In some examples, the radar sub-system 134 may be a radar-on-a-chip, a radar transceiver, receive/transmit antenna(s), RF front end circuitry, RF processing circuitry, filters, and/or digital signal processing circuitry integrated on a single ship. The radar sub-system may operate at 60 GHz range (e.g., 58.0 to 63.5 GHz with a bandwidth of 5.5 GHz). Such a radar sub-system may provide millimeter level spatial resolution (with signal processing enhancements) at low power consumption levels compared to a camera on the near-eye display device 120. The radar sub-system 134 may be used standalone to detect hand or finger gestures, in combination with a camera to activate the camera upon detecting motion or for enhanced gesture detection, and to obtain 3D information, which may be combined with 2D image information from the camera to reconstruct 3D scenes.

In some examples, the input/output interface 140 may be a device that allows a user to send action requests to the optional console 110. As used herein, an "action request" may be a request to perform a particular action. For example, an action request may be to start or to end an application or to perform a particular action within the application. The input/output interface 140 may include one or more input devices. Example input devices may include a keyboard, a mouse, a game controller, a glove, a button, a touch screen, or any other suitable device for receiving action requests and communicating the received action requests to the optional console 110. In some examples, an action request received by the input/output interface 140 may be communicated to the optional console 110, which may perform an action corresponding to the requested action.

In some examples, the optional console 110 may provide content to the near-eye display device 120 for presentation to the user in accordance with information received from one or more of external imaging device 150, the near-eye display device 120, and the input/output interface 140. For example, in the example shown in Figure 1, the optional console 110 may include an application store 112, a headset tracking module 114, a virtual reality engine 116, and an eye tracking module 118. Some examples of the optional console 110 may include different or additional modules than those described in conjunction with Figure 1. Functions further described below may be distributed among components of the optional console 110 in a different manner than is described here.

In some examples, the optional console 110 may include a processor and a non-transitory computer-readable storage medium storing instructions executable by the processor. The processor may include multiple processing units executing instructions in parallel. The non-transitory computer-readable storage medium may be any memory, such as a hard disk drive, a removable memory, or a solid-state drive (e.g., flash memory or dynamic random access memory (DRAM)). In some examples, the modules of the optional console 110 described in conjunction with Figure 1 may be encoded as instructions in the non-transitory computer-readable storage medium that, when executed by the processor, cause the processor to perform the functions further described below. It should be appreciated that the optional console 110 may or may not be needed or the optional console 110 may be integrated with or separate from the near-eye display device 120.

In some examples, the application store 112 may store one or more applications for execution by the optional console 110. An application may include a group of instructions that, when executed by a processor, generates content for presentation to the user. Examples of the applications may include gaming applications, conferencing applications, video playback application, or other suitable applications.

In some examples, the virtual reality engine 116 may execute applications within the artificial reality system environment 100 and receive position information of the near-eye display device 120, acceleration information of the near-eye display device 120, velocity information of the near-eye display device 120, predicted future positions of the near-eye display device 120, or any combination thereof from the headset tracking module 114. In some examples, the virtual reality engine 116 may also receive estimated eye position and orientation information from the eye tracking module 118. Based on the received information, the virtual reality engine 116 may determine content to provide to the near-eye display device 120 for presentation to the user.

In some examples, the eye tracking module 118, which may be implemented as a processor, may receive eye tracking data from the eye tracking unit 130 and determine the position of the user's eye based on the eye tracking data. In some examples, the position of the eye may include an eye's orientation, location, or both relative to the near-eye display device 120 or any element thereof. So, in these examples, because the eye's axes of rotation change as a function of the eye's location in its socket, determining the eye's location in its socket may allow the eye tracking module 118 to more accurately determine the eye's orientation.

In some examples, a location of a projector of a display system may be adjusted to enable any number of design modifications. For example, in some instances, a projector may be located in front of a viewer's eye (i.e., "front-mounted" placement). In a front-mounted placement, in some examples, a projector of a display system may be located away from a user's eyes (i.e., "world-side"). In some examples, a head-mounted display (HMD) device may utilize a front-mounted placement to propagate light towards a user's eye(s) to project an image.

Figure 2 illustrates a perspective view of a near-eye display device in the form of a head-mounted display (HMD) device 200, according to an example. In some examples, the head-mounted device (HMD) device 200 may be a part of a virtual reality (VR) system, an augmented reality (AR) system, a mixed reality (MR) system, another system that uses displays or wearables, or any combination thereof. In some examples, the head-mounted display (HMD) device 200 may include a body 220 and a head strap 230. Figure 2 shows a bottom side 223, a front side 225, and a left side 227 of the body 220 in the perspective view. In some examples, the head strap 230 may have an adjustable or extendible length. In particular, in some examples, there may be a sufficient space between the body 220 and the head strap 230 of the head-mounted display (HMD) device 200 for allowing a user to mount the head-mounted display (HMD) device 200 onto the user's head. For example, the length of the head strap 230 may be adjustable to accommodate a range of user head sizes. In some examples, the head-mounted display (HMD) device 200 may include additional, fewer, and/or different components.

In some examples, the head-mounted display (HMD) device 200 may present, to a user, media or other digital content including virtual and/or augmented views of a physical, real-world environment with computer-generated elements. Examples of the media or digital content presented by the head-mounted display (HMD) device 200 may include images (e.g., two-dimensional (2D) or three-dimensional (3D) images), videos (e.g., 2D or 3D videos), audio, or any combination thereof. In some examples, the images and videos may be presented to each eye of a user by one or more display assemblies (not shown in Figure 2) enclosed in the body 220 of the head-mounted display (HMD) device 200.

In some examples, the head-mounted display (HMD) device 200 may include various sensors (not shown), such as depth sensors, motion sensors, position sensors, and/or eye tracking sensors. Some of these sensors may use any number of structured or unstructured light patterns for sensing purposes. In some examples, the head-mounted display (HMD) device 200 may include an input/output interface 140 for communicating with a console 110, as described with respect to Figure 1. In some examples, the head-mounted display (HMD) device 200 may include a virtual reality engine (not shown), but similar to the virtual reality engine 116 described with respect to Figure 1, that may execute applications within the head-mounted display (HMD) device 200 and receive depth information, position information, acceleration information, velocity information, predicted future positions, or any combination thereof of the head-mounted display (HMD) device 200 from the various sensors.

In some examples, the information received by the virtual reality engine 116 may be used for producing a signal (e.g., display instructions) to the one or more display assemblies. In some examples, the head-mounted display (HMD) device 200 may include locators (not shown), but similar to the locators 126 described in Figure 1, which may be located in fixed positions on the body 220 of the head-mounted display (HMD) device 200 relative to one another and relative to a reference point. Each of the locators may emit light that is detectable by an external imaging device. This may be useful for the purposes of head tracking or other movement/orientation. It should be appreciated that other elements or components may also be used in addition or in lieu of such locators.

It should be appreciated that in some examples, a projector mounted in a display system may be placed near and/or closer to a user's eye (i.e., "eye-side"). In some examples, and as discussed herein, a projector for a display system shaped liked eyeglasses may be mounted or positioned in a temple arm (i.e., a top far corner of a lens side) of the eyeglasses. It should be appreciated that, in some instances, utilizing a back-mounted projector placement may help to reduce size or bulkiness of any required housing required for a display system, which may also result in a significant improvement in user experience for a user.

In some examples, the head-mounted display (HMD) device 200 may include a radar sub-system 218, which may operate at 60 GHz range (e.g., 58.0 to 63.5 GHz with a bandwidth of 5.5 GHz). The radar sub-system 218 may provide millimeter level spatial resolution (with signal processing enhancements) at low power consumption levels compared to the camera 215 on the head-mounted display (HMD) device 200. The radar sub-system 218 may be used standalone to detect hand or finger gestures, in combination with the camera 215 to activate the camera upon detecting motion or for enhanced gesture detection, and to obtain 3D information, which may be combined with 2D image information from the camera 215 to reconstruct 3D scenes.

Figure 3A is a perspective view 300A of a near-eye display device 300 in the form of a pair of glasses (or other similar eyewear), according to an example. In some examples, the near-eye display device 300 may be a specific example of near-eye display device 120 of Figure 1 and may be configured to operate as a virtual reality display, an augmented reality (AR) display, and/or a mixed reality (MR) display.

In some examples, the near-eye display device 300 may include a frame 305 and a display 310. In some examples, the display 310 may be configured to present media or other content to a user. In some examples, the display 310 may include display electronics and/or display optics, similar to components described with respect to Figures 1-2. For example, as described above with respect to the near-eye display device 120 of Figure 1, the display 310 may include a liquid crystal display (LCD) display panel, a light-emitting diode (LED) display panel, or an optical display panel (e.g., a waveguide display assembly). In some examples, the display 310 may also include any number of optical components, such as waveguides, gratings, lenses, mirrors, etc. In other examples, the display 210 may include a projector, or in place of the display 310 the near-eye display device 300 may include a projector.

In some examples, the near-eye display device 300 may further include various sensors 350a, 350b, 350c, 350d, and 350e on or within a frame 305. In some examples, the various sensors 350a-350e may include any number of depth sensors, motion sensors, position sensors, inertial sensors, and/or ambient light sensors, as shown. In some examples, the various sensors 350a-350e may include any number of image sensors configured to generate image data representing different fields of views in one or more different directions. In some examples, the various sensors 350a-350e may be used as input devices to control or influence the displayed content of the near-eye display device, and/or to provide an interactive virtual reality (VR), augmented reality (AR), and/or mixed reality (MR) experience to a user of the near-eye display device 300. In some examples, the various sensors 350a-350e may also be used for stereoscopic imaging or other similar application.

In some examples, the near-eye display device 300 may further include one or more illuminators 330 to project light into a physical environment. The projected light may be associated with different frequency bands (e.g., visible light, infra-red light, ultra-violet light, etc.), and may serve various purposes. In some examples, the one or more illuminator(s) 330 may be used as locators, such as the one or more locators 126 described above with respect to Figures 1-2.

In some examples, the near-eye display device 300 may also include a camera 315 or other image capture unit. The camera 315, for instance, may capture images of the physical environment in the field of view. In some instances, the captured images may be processed, for example, by a virtual reality engine (e.g., the virtual reality engine 116 of Figure 1) to add virtual objects to the captured images or modify physical objects in the captured images, and the processed images may be displayed to the user by the display 310 for augmented reality (AR) and/or mixed reality (MR) applications. The near-eye display device 300 may also include eye tracking sensors 312.

In some examples, the near-eye display device 300 may include a radar sub-system 318, which may operate at 60 GHz range (e.g., 58.0 to 63.5 GHz with a bandwidth of 5.5 GHz). The radar sub-system 318 may provide millimeter level resolution (with signal processing enhancements) at low power consumption levels compared to the camera 315 on the near-eye display device 300. The radar sub-system 318 may be used standalone to detect hand or finger gestures, in combination with the camera 315 to activate the camera upon detecting motion or for enhanced gesture detection, and to obtain 3D information, which may be combined with 2D image information from the camera 315 to reconstruct 3D scenes.

Figure 3B is a top view 300B of a near-eye display device 300 in the form of a pair of glasses (or other similar eyewear), according to an example. In some examples, the near-eye display device 300 may include a frame 305 having a form factor of a pair of eyeglasses. The frame 305 supports, for each eye: a fringe projector 314 such as any fringe projector variant considered herein, a display 310 to present content to an eye box 366, eye tracking sensors 312, and one or more illuminators 330. The illuminators 330 may be used for illuminating an eye box 366, as well as, for providing glint illumination to the eye. A fringe projector 314 may provide a periodic fringe pattern onto a user's eye. The display 310 may include a pupil-replicating waveguide to receive the fan of light beams and provide multiple laterally offset parallel copies of each beam of the fan of light beams, thereby extending a projected image over the eye box 366.

In some examples, the pupil-replicating waveguide may be transparent or translucent to enable the user to view the outside world together with the images projected into each eye and superimposed with the outside world view. The images projected into each eye may include objects disposed with a simulated parallax, so as to appear immersed into the real-world view.

In some examples, the image processing and eye position/orientation determination functions may be performed by a central controller, not shown, of the near-eye display device 300. The central controller may also provide control signals to the display 310 to generate the images to be displayed to the user, depending on the determined eye positions, eye orientations, gaze directions, eyes vergence, etc.

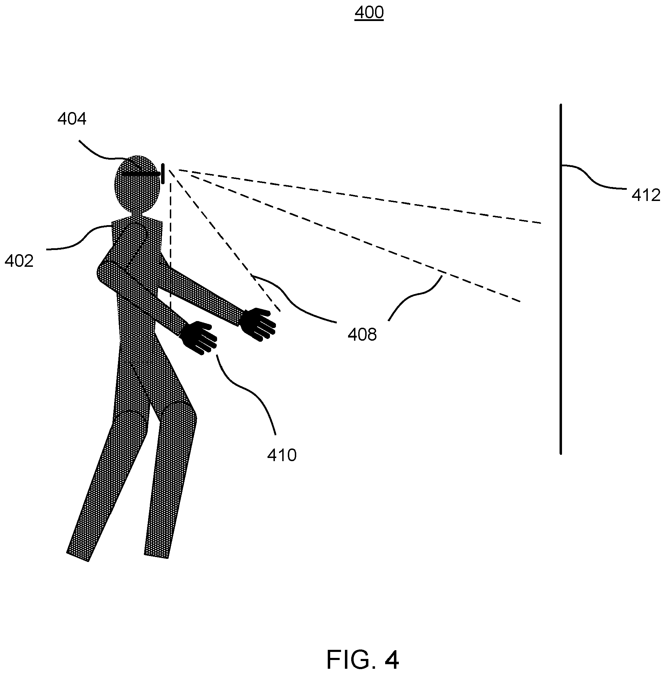

Figure 4 illustrates direct or indirect gesture detection through a radar on a near-eye display device, according to examples. Diagram 400 shows a user 402 wearing a near-eye display device 404 (e.g., a head-mounted display (HMD) device). The user 402 may interact with the displayed augmented reality (AR) / virtual reality (VR) content through gesture detection 408 of hand and/or finger gestures 410. The gesture detection 408 may be direct or indirect from a nearby reflective surface 412.

In some examples, the near-eye display device 404 (head-mounted display (HMD) device) may include a camera and a radar sub-system. The radar sub-system may detect hand/finger gestures 410 using radar waves and detecting motion through time difference and frequency shift measurements. To allow for natural poses for the user 402 (not to have them put their hands up against the near-eye display device 404 and make gestures with hands on their lap, etc.), the radar sub-system may be positioned on the near-eye display device 404 such that the radar antennas face downward at an angle. The radar sub-system may also detect body motion (arm, head, torso, leg, etc.), in some cases.

In other examples, the radar sub-system may be used in combination with the outward facing camera on the near-eye display device 404 for more accurate gesture detection, where outputs of the radar sub-system and the camera may be combined in processing captured gestures. Furthermore, the radar sub-system (and the camera) may also detect gestures indirectly from a reflective surface 412. Additionally, the radar sub-system, which consumes less power compared to the camera may be maintained in active mode longer while the camera is turned off, and upon detecting motion (e.g., hand motion), the radar sub-system may be used to turn the camera on and detect gestures through the camera.

In yet other examples, the camera may capture a 2D image of the real scene around the user 402. The radar sub-system may obtain depth information associated with the real scene, and the 2D image and the depth information may be combined by a processor to reconstruct a 3D image of the real scene (for augmented reality (AR) / virtual reality (VR) applications).

In further examples, the radar sub-system may be used to detect bio-signals of the user 402 such as breath rate, heart rate, hand or finger tremors or twitching, and provide the results to the processor for health monitoring. The processor may forward the detected bio-signals to a health monitoring server, for example, the server of a health care provider. The radar sub-system and the camera together may be referred to as a detection system of the near-eye display device detecting (and capturing) gestures, 3D characteristics of a scene around the user, 2D images of the scene, and/or bio-signals of the user.

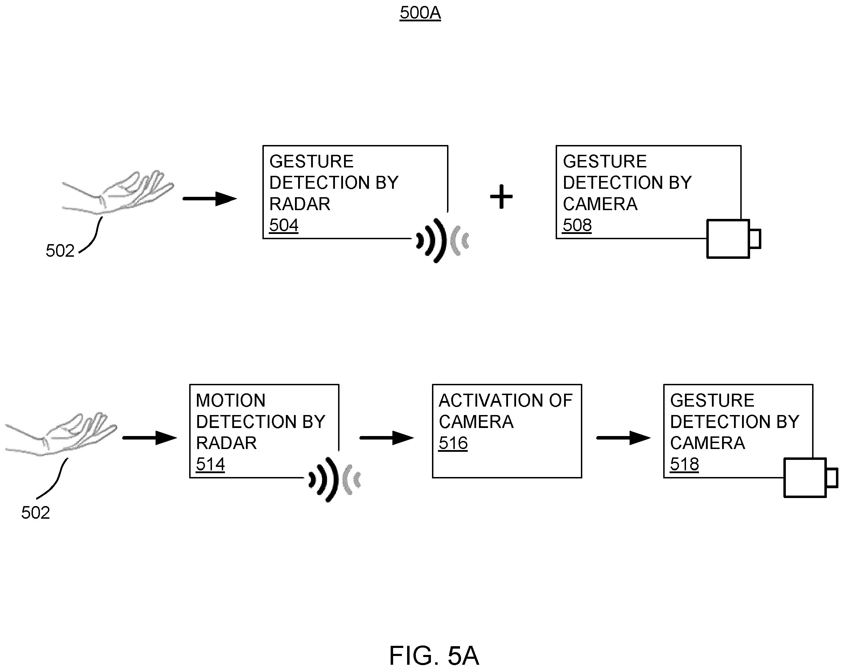

Figure 5A illustrates enhanced gesture detection through a combination of a radar and a camera, and activation of a gesture detecting camera through a radar in a near-eye display device, according to examples. Diagram 500A shows a gesture 502 (hand or finger gesture) being detected by a radar sub-system (504) or, alternatively, by a combination of radar sub-system (504) and camera (508) for enhanced accuracy. The diagram also shows the gesture 502 being detected by the camera (518), where the camera is activated (516) upon detection of a motion (hand or finger) by the radar sub-system (514).

In some examples, a radar-on-a-chip may be integrated with a near-eye display device with the radar antenna(s) facing downward at an angle to allow users present their gestures in a natural pose. The radar sub-system may employ mm-wave (60 GHz) frequency range, which may allow resolutions down to millimeter level (with signal processing improvements). As the radar sub-system consumes substantially less power compared to the camera, the radar sub-system may be kept in active mode for longer periods (e.g., as long as the near-eye display device is worn) and used to activate the camera as shown in the second scenario in the diagram 500A.

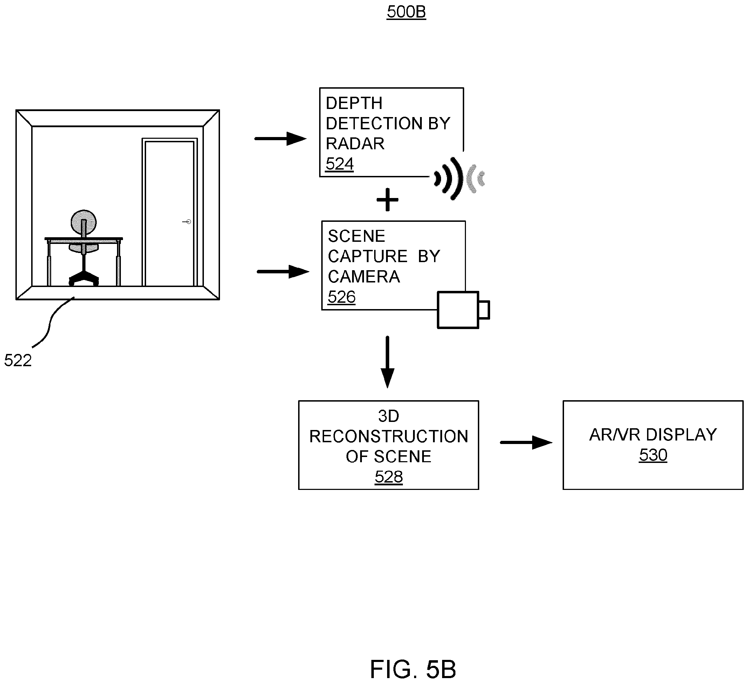

Figure 5B illustrates 3D reconstruction of a real scene through a combination of a camera and a radar in a near-eye display device, according to examples. Diagram 500B shows a real scene 522 being captured (2D image) by a camera 526 and depth information associated with the scene 522 being captured by the radar sub-system (524). The depth information and the 2D image information may be combined for a 3D reconstruction of the scene (528), which may be presented to the user through the augmented reality (AR) / virtual reality (VR) display (530).

In some examples, the radar sub-system may capture depth information for larger portions or an entirety of the scene 522 and a full 3D reconstruction of the scene 522 may be created. In other examples, the radar sub-system may focus on a small portion of the scene and assist in 3D motions associated with the scene. For example, the scene may include a cup and the user may grab and move the cup (e.g., drinking motion). The radar sub-system may track the cup's motion and that information may be used to accurately recreate the cup (and its motion) in a virtual reconstruction of the scene.

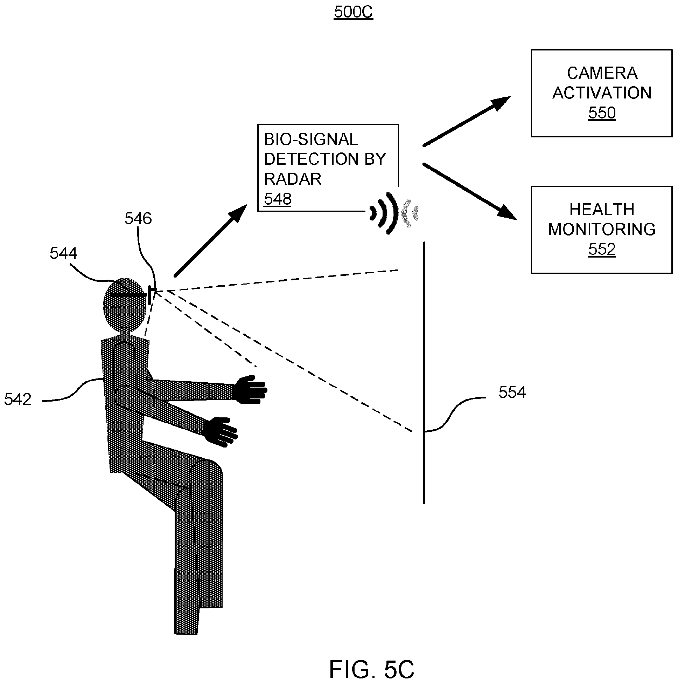

Figure 5C illustrates bio-signal detection by a radar in a near-eye display device for camera activation or health monitoring, according to examples. Diagram 500C shows a user 542 wearing a near-eye display device 544 with a radar sub-system 546. The radar sub-system 546 may be used for bio-signal detection (548) directly or indirectly from a reflective surface 554. The radar sub-system's detection of the bio-signal may be used for health monitoring 552 and/or to activate the camera (550) to capture the moment.

In some examples, the radar sub-system 546 may be used to detect bio-signal(s) of the user such as breath rate, heart rate, tremors or twitching of hands or fingers, etc. The detection may be used for health monitoring (552), where captured bio-signals may be stored and/or processed locally or transmitted by the near-eye display device to a server (e.g., a health care provider's server). In other examples, the radar sub-system 546 may capture a bio-signal, which may be over a predefined threshold (e.g., breath rate), and activate the camera to capture "the moment". For example, if the user's breath rate suddenly increases, an image (or video) of the environment may provide valuable context to the user or a health care provider.

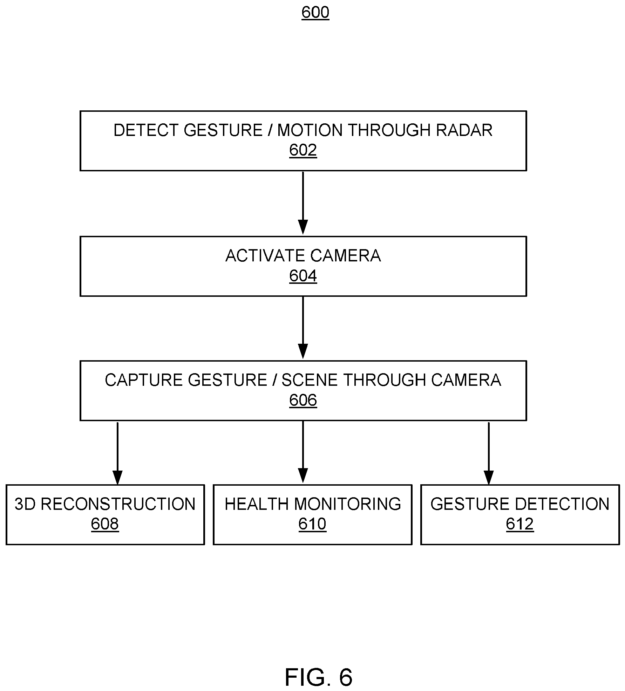

Figure 6 illustrates a flow diagram for a method of gesture and 3D scene capture and detection through a radar and/or a camera on a near-eye display device, according to some examples. The method 600 is provided by way of example, as there may be a variety of ways to carry out the method described herein. Although the method 600 is primarily described as being performed by the components of Figures 2-3B, the method 600 may be executed or otherwise performed by one or more processing components of another system or a combination of systems. Each block shown in Figure 6 may further represent one or more processes, methods, or subroutines, and one or more of the blocks (e.g., the selection process) may include machine readable instructions stored on a non-transitory computer readable medium and executed by a processor or other type of processing circuit to perform one or more operations described herein.

At block 602, a hand/finger gesture or a motion of hand/finger may be detected by a radar sub-system of a near-eye display device. The radar sub-system may employ mm-waves (e.g., 60 GHz) with a wide band (e.g., 5.5 GHz) allowing millimeter level resolution. Antenna(s) of the radar sub-system may be positioned such that hands / fingers of the user can be detected in a natural pose. Gesture(s) detected by the radar sub-system may be used as input for one or more applications executed on the near-eye display device.

At block 604, an outward facing camera of the near-eye display device may be activated upon detection of a hand or finger motion by the radar sub-system. As mentioned herein, the radar sub-system consumes substantially less power compared to the camera. Thus, the radar sub-system may be kept active for longer periods while the camera is turned off and activated upon detection of the motion by the radar sub-system.

At block 606, the gesture or a scene around the user may be captured by the activated camera. Gestures may be detected by a combination of the camera and the radar sub-system for enhanced accuracy as shown in block 612. In some examples, a 2D image of the scene captured by the camera may be complemented by depth information captured by the radar sub-system in order to reconstruct a 3D image of the scene as shown in block 608. Furthermore, the radar sub-system may capture health information through bio-signals such as breath rate, heart rate, etc., which may be used for health monitoring as shown in block 610.

According to examples, a method of making a near-eye display device with a radar and a camera is described herein. A system of making the near-eye display device is also described herein. A non-transitory computer-readable storage medium may have an executable stored thereon, which when executed instructs a processor to perform the methods described herein.

In the foregoing description, various examples are described, including devices, systems, methods, and the like. For the purposes of explanation, specific details are set forth in order to provide a thorough understanding of examples of the disclosure. However, it will be apparent that various examples may be practiced without these specific details. For example, devices, systems, structures, assemblies, methods, and other components may be shown as components in block diagram form in order not to obscure the examples in unnecessary detail. In other instances, well-known devices, processes, systems, structures, and techniques may be shown without necessary detail in order to avoid obscuring the examples.

The figures and description are not intended to be restrictive. The terms and expressions that have been employed in this disclosure are used as terms of description and not of limitation, and there is no intention in the use of such terms and expressions of excluding any equivalents of the features shown and described or portions thereof. The word "example" is used herein to mean "serving as an example, instance, or illustration." Any embodiment or design described herein as "example' is not necessarily to be construed as preferred or advantageous over other embodiments or designs.

Although the methods and systems as described herein may be directed mainly to digital content, such as videos or interactive media, it should be appreciated that the methods and systems as described herein may be used for other types of content or scenarios as well. Other applications or uses of the methods and systems as described herein may also include social networking, marketing, content-based recommendation engines, and/or other types of knowledge or data-driven systems.

## Claims

1. A detection system for a near-eye display device, comprising:
a radar sub-system configured to detect a gesture, wherein the radar sub-system comprises at least one antenna, a radio frequency (RF) front end, RF processing circuitry, or digital processing circuitry;
an image sensor configured to capture a two-dimensional (2D) image of the detected gesture; and
a processor communicatively coupled to the radar sub-system and the image sensor, the processor to:
activate the image sensor upon detection of the gesture by the radar sub-system; and
provide an input to an application executed at the near-eye display device based on the detected gesture.

2. The detection system of claim 1, wherein the gesture comprises at least one of a hand gesture, a finger gesture, or a body gesture.

3. The detection system of claim 1 or claim 2 and one or more of the following:
wherein the radar sub-system is configured to detect the gesture directly or indirectly from a nearby reflective surface;
wherein the radar sub-system comprises a millimeter-wave radar sub-system.

4. The detection system of any preceding claim, wherein an antenna of the radar sub-system is positioned downward from a head of a user and at a predefined angle to detect the gesture while the user is in a natural pose.

5. The detection system of any preceding claim, wherein the image sensor comprises an outward-facing camera configured to capture the 2D image of the gesture directly or indirectly from a nearby reflective surface.

6. The detection system of any preceding claim, wherein the processor is further configured to:
compare an output of the radar sub-system and an output of the image sensor so as to enhance an accuracy of the detected gesture.

7. A detection system for a near-eye display device, comprising:
a radar sub-system configured to capture a depth characteristic of a scene around a user wearing the near-eye display device, wherein the radar sub-system comprises at least one antenna, a radio frequency (RF) front end, RF processing circuitry, or digital processing circuitry;
an image sensor configured to capture a two-dimensional (2D) image of the scene; and
a processor communicatively coupled to the radar sub-system and the image sensor, the processor to:
receive the 2D image from the image sensor and the depth characteristic from the radar sub-system; and
reconstruct a three-dimensional (3D) image of the scene based on the 2D image and the depth characteristic.

8. The detection system of claim 7, wherein the depth characteristic comprises depth information associated with at least one object in the scene.

9. The detection system of claim 7 or claim 8, wherein the radar sub-system comprises a millimeter-wave radar sub-system that provides millimeter level spatial resolution;
optionally, wherein the radar sub-system employs 58.0 GHz to 63.5 GHz frequency range with a bandwidth of 5.5 GHz.

10. The detection system of any one of claims 7 to 9, wherein the processor is further configured to provide the reconstructed 3D image of the scene to a display of the near-eye display device.

11. A detection system for a near-eye display device, comprising:
a radar sub-system configured to detect a bio-signal of a user wearing the near-eye display device, wherein the radar sub-system comprises at least one antenna, a radio frequency (RF) front end, RF processing circuitry, or digital processing circuitry; and
a processor communicatively coupled to the radar sub-system, the processor to:
provide the detected bio-signal to a health monitoring server.

12. The detection system of claim 11, wherein the bio-signal comprises at least one of a breath rate, a heart rate, a twitch, or a tremor.

13. The detection system of claim 12, further comprising:
an image sensor configured to capture an image, wherein the processor is further configured to:
activate the image sensor upon detection of the bio-signal by the radar sub-system; and
instruct the image sensor to capture the image;

14. The detection system of any one of claims 11 to 13, wherein the image sensor comprises an outward-facing camera configured to capture the image directly or indirectly from a nearby reflective surface;
optionally, wherein the captured image comprises a scene around the user or an image of the user.

15. The detection system of any one of claims 11 to 14 and one or more of the following:
wherein an antenna of the radar sub-system is positioned downward from a head of the user and at a predefined angle to detect the bio-signal while the user is in a natural pose;
wherein an antenna of the radar sub-system is positioned to detect the bio-signal indirectly from a nearby reflective surface;
wherein the radar sub-system comprises a millimeter-wave radar sub-system.
